Europäisches Patentamt

European Patent Office

Office européen des brevets

(19)

(11) Veröffentlichungsnummer: **0 265 724 B1**

# EUROPÄISCHE PATENTSCHRIFT

(12)

(45) Veröffentlichungstag der Patentschrift: **08.05.91**

(51) Int. Cl.⁵: **F03G 1/06, A61M 5/14**

(21) Anmeldenummer: **87114624.7**

(22) Anmeldetag: **07.10.87**

(54) Federaufzugsgetriebe für das Laufwerk eines Gerätes mit Durchlaufsicherung.

(30) Priorität: **30.10.86 DE 3636948**

(43) Veröffentlichungstag der Anmeldung:
**04.05.88 Patentblatt 88/18**

(45) Bekanntmachung des Hinweises auf die
Patenterteilung:
**08.05.91 Patentblatt 91/19**

(84) Benannte Vertragsstaaten:
**AT BE CH DE ES FR GB GR IT LI LU NL SE**

(56) Entgegenhaltungen:
**EP-A- 0 167 318**
**WO-A-85/01443**
**FR-A- 2 339 789**

(73) Patentinhaber: **B. Braun Melsungen AG**
**Carl-Braun Strasse**
**W-3508 Melsungen(DE)**

(72) Erfinder: **Dold, Michael Robert**
**Gerwig-Strasse 16**
**W-7741 Schönwald(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

## Beschreibung

Die Erfindung bezieht sich auf ein Federaufzugsgetriebe für das Laufwerk eines Gerätes, insbesondere eines medizinischen Infusionsgerätes, nach dem Oberbegriff des Hauptanspruches.

Federaufzugsgetriebe werden überall dort eingesetzt, wo ohne Stromquelle mechanisch ein Teil über einen längeren Zeitraum gleichmäßig bewegt werden muß. Dies trifft auf Uhren aller Art zu. Ferner dient bei Geräten für Dauerinfusionen ein Federmechanismus des Laufwerkes zum Vorschub des Spritzenstößels einer Injektionsspritze, wobei der Vorschub von einem mechanischen Uhrwerk gesteuert wird. Ein solches Gerät für Dauerinfusionen ist in DE-PS 29 06 830 beschrieben. Allen Federaufzugsgetrieben oder Uhrwerksantrieben ist der Mangel gemeinsam, daß das Laufwerk in kürzester Zeit unkontrolliert abläuft, wenn irgendein Teil zwischen dem Federhaus und dem Gangregler bricht. Dies ist darauf zurückzuführen, daß sich die Spiralfeder frei entspannen kann, sobald sie von dem Gangregler entkoppelt ist und daß sie dabei die gespeicherte Energie auf einmal freigibt. Diese Erscheinung ist bei jeder Einsatzart eines Federaufzugsgetriebes natürlich unerwünscht; bei einem Gerät für Dauerinfusionen oder einem ähnlichen medizinischen Apparat ist sie jedoch gefährlich, weil die in der Injektionsspritze enthaltene hochwirksame körpereigene chemische Substanz bzw. ein Arzneimittel plötzlich und vollständig in die Blutbahn eines Patienten injiziert wird, anstatt über einen längeren Zeitraum kontinuierlich fein dosiert abgegeben zu werden.

Zur Behebung des Nachteiles dieses Federaufzugsgetriebes wurde das eingangs erwähnte Federaufzugsgetriebe gemäß EP-A-167 318 geschaffen, das mit einer Sperre ausgerüstet ist, die ein unkontrolliertes Durchlaufen der Abtriebswelle bei einem Defekt in dem Getriebe verhindern soll. Dieses Federaufzugsgetriebe arbeitet aus Sicherheitsgründen mit zwei zueinander umfangsmäßig versetzten Antriebsrädern, die mit zwei zugeordneten Ritzeln auf der Abtriebswelle kämmen und zusätzlich trägt die Abtriebswelle ein Kraftübertragungsrad mit wenigen Zähnen, das mit einem zylindrischen Teil mit einer einzigen Kerbe zusammenwirkt, der auf einer dritten Welle sitzt. Diese Vielzahl von Getriebegliedern macht das bekannte Federaufzugsgetriebe aufwendig und teuer, ohne daß die Sicherheitsvorrichtung zufriedenstellend funktioniert. Die Sperre ist nämlich nur dann wirksam, wenn der Defekt in der ersten Getriebestufe auftritt - Defekte in den nachgeordneten Getriebestufen wirken sich nicht auf die Sperrvorrichtung aus, d.h. sie verursachen keine Blockierung der Abtriebswelle und ein unbeabsichtigtes spontanes Entleeren der mit dem Federaufzugsgetriebe gekoppelten Injektionsspritze bleibt durchaus möglich.

Der Erfindung liegt die Aufgabe zugrunde, Federaufzugsgetriebe mit einer Sicherheitseinrichtung gegen unkontrolliertes Durchlaufen der Abtriebswelle bei Unterbrechung der Wirkverbindung zwischen Spiralfeder und Gangregler auszustatten.

Diese Aufgabe wird bei einem Federaufzugsgetriebe der gattungsgemäßen Art dadurch gelöst, daß das drehbare Sperrelement von einem beweglichen Trägerteil in Ausrückstellung zu dem ersten Kraftübertragungsrad gehalten ist, und daß an dem Trägerteil ein zweites Kraftübertragungsrad angeordnet ist, das mit dem ersten Kraftübertragungsrad kämmt und das Trägerteil zur Freigabe des Sperrelementes bei erhöhter Drehzahl bzw. Beschleunigung des ersten Kraftübertragungsrades tangential zu diesem auslenkt.

Die Erfindung schafft also ein Federaufzugsgetriebe mit einer Sicherheitsvorrichtung, die auf sämtliche Defekte eines Getriebes zwischen Spiralfeder und Gangregler mit der Blockierung der Abtriebswelle reagiert. Auf diese Weise wird ein einfach aufgebautes Federaufzugsgetriebe mit hoher Sicherheitsfunktion erzielt, das weniger zusätzlicher kosten- und platzaufwendiger Konstruktionsmaßnahmen bedarf, um die Funktionszuverlässigkeit zu steigern.

Die Gemeinsamkeit dieser Merkmale schafft eine Sicherheitseinrichtung bei einem Federaufzugsgetriebe, die bei Bruch, z.B. einer Achse, das freie Durchlaufen der Abtriebswelle dadurch verhindert, daß das Sperrelement in das mit der Abtriebswelle verbundene Kraftübertragungsrad eingreift und dieses solange gegen Verdrehen sperrt, bis das Sperrelement bewußt in seine Ausrückstellung zurückgeführt wird, und das bewegliche Trägerteil seine Halteposition für das Sperrelement wieder eingenommen hat. Das mit dem ersten Kraftübertragungsrad zusammenwirkende zweite Kraftübertragungsrad an dem beweglichen Trägerteil bildet eine Bremse, die der Bewegung des Kraftübertragungsrades an der Abtriebswelle entgegenwirkt, wenn sich durch einen Fehler im Federaufzugsgetriebe die Drehzahl des Kraftübertragungsrades erhöht und sich an dem zweiten Kraftübertragungsrad ein Drehmoment aufbaut. Durch das Drehmoment an dem zweiten Kraftübertragungsrad erzeugte Tangentialkräfte drücken das Trägerteil tangential von dem ersten Kraftübertragungsrad an der Abtriebswelle weg und das seines Haltes beraubte drehbare Sperrelement fällt gegen den Umfang des ersten Kraftübertragungsrades und sperrt das Getriebe gegen weiteres ungewolltes freies Durchlaufen. Bei Anwendung des in erfindungsgemäßer Weise gesicherten Federaufzugsgetriebes auf ein Gerät für Dauerinfusionen wird ein unbeabsichtigtes spontanes Entleeren der Injektionsspritze bei Getriebedefekten zuverlässig verhindert, d.h. es wird sichergestellt, daß die zu applizierende Flüssigkeit infolge einer durchlaufenden Abtriebswelle nicht plötzlich in den Patienten gepumpt wird.

Die Kraftübertragungsräder können Zahnräder (Klinkenräder) oder Reibräder sein. Im ersten Falle wird als Sperrelement eine Sperrklinke benutzt, während im zweiten Falle eine Bremsbacke als Sperrelement dient.

In vorteilhafter Ausgestaltung der Erfindung ist vorgesehen, daß das Sperrelement und das Trägerteil federbelastet sind. An das Sperrelement greift eine Zugfeder an, die es gegen das in Halteposition befindliche Trägerteil angezogen hält und veranlaßt, daß das Sperrelement bei Entfernung des Trägerteils aus seiner Halteposition gegen den Umfang des ersten Kraftübertragungsrades auf der Abtriebswelle angeschwenkt wird. Das Trägerteil steht unter der Wirkung einer Feder, z.B. einer Biegefeder, die es gegen einen Anschlag in die Halteposition drückt. Die Kraft der Feder ist abgestimmt auf das Drehmoment des Getriebegliedes, bei dem das Trägerteil das Sperrelement freigeben soll.

Ein weiterer wesentlicher Vorteil der Erfindung besteht in der Testmöglichkeit der Durchlaufsicherung zur Erkennung eventuell vorliegender Funktionsstörungen durch Zurückdrehen des Aufziehers. Da das Funktionsgetriebe zweckmäßigerweise durch eine Rutschkupplung mit der Feder verbunden ist, ist es möglich, durch Überwindung des Drehmomentes, welches die Rutschkupplung überträgt, die Durchlaufsicherung von Hand zu aktivieren. Auf diese Weise wird der Einsatz der Durchlaufsicherung im Bereich der Medizintechnik ermöglicht.

In vorteilhafter Ausgestaltung der Erfindung ist vorgesehen, daß das zweite Kraftübertragungsrad mit mindestens einem Übersetzungselement zusammenwirkt. Als Übersetzungselement kann ein weiteres Kraftübertragungsrad und/oder ein Flügelrad bzw. ein Schwungrad an dem Trägerteil vorgesehen sein. Das Übersetzungselement dient der Erhöhung des Drehmomentes an dem zweiten Kraftübertragungsrad, falls besonders niedrige Abtriebsgeschwindigkeiten vorhanden sind oder bei geringsten Drehzahländerungen gestoppt werden soll. Das Flügelrad und das Schwungrad sind gut geeignet, hohe Gegenkräfte zu erzeugen, wobei das Flügelrad geschwindigkeitsabhängig ist und eine Abbremsung des ersten Kraftübertragungsrades auf der Abtriebswelle bei höherer Drehzahl bewirkt, während das Schwungrad beschleunigungsabhängig ist und infolge seiner Trägheit bei Geschwindigkeitsänderungen sofort einen hohen Widerstand gegen die Drehung des ersten Kraftübertragungsrades auf der Abtriebswelle erzeugt.

Das Trägerteil kann als drehbare Schwinge ausgebildet sein. Die Schwinge wird von dem an dem zweiten Kraftübertragungsrad aufgebauten Drehmoment um ihren Drehpunkt so gekippt, daß sie sich aus der Reichweite des Sperrelementes herausbewegt und diese zum Angriff an das Kraftübertragungsrad auf der Abtriebswelle freigibt. Auch kann das Trägerteil als tangential zum ersten Kraftübertragungsrad verstellbarer Schlitten ausgebildet sein, der unter dem Einfluß des beschleunigt angetriebenen zweiten Kraftübertragungsrades tangential weggeführt wird.

In der Zeichnung ist ein Ausführungsbeispiel der Erfindung schematisch dargestellt. Es zeigen:

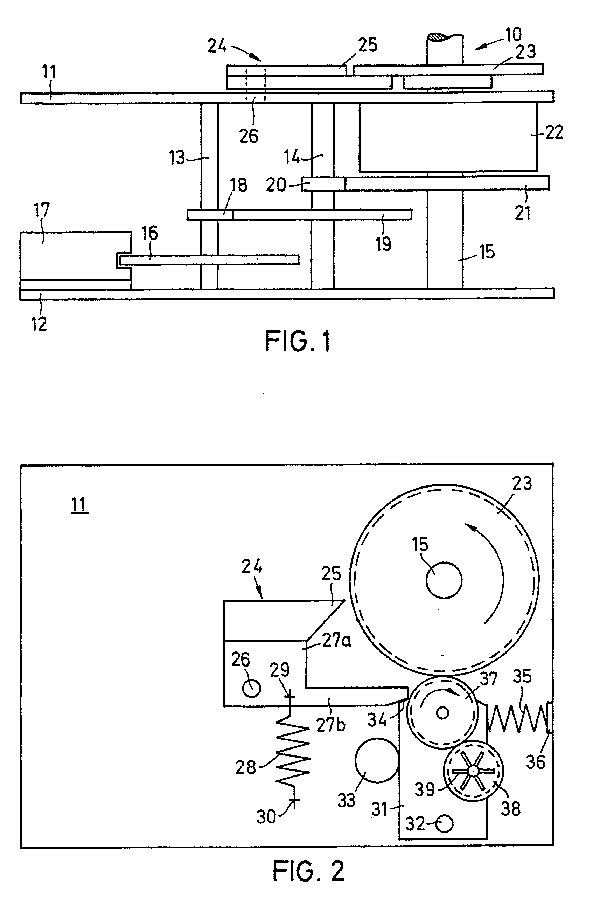

Fig. 1 eine Seitenansicht eines Federaufzugsgetriebes,

Fig. 2 eine Draufsicht auf das Federaufzugsgetriebe nach Fig. 1 in ungesperrtem Zustand, und

Fig. 3 die Anordnung nach Fig. 2 in gesperrtem Zustand.

Ein ganggeregeltes Federaufzugsgetriebe 10, beispielsweise für ein Dauerinfusionsgerät, ist mit mindestens zwei im Abstand befindlichen Deckplatten 11,12 ausgestattet, in denen die Enden von mindestens zwei Achsen 13, 14 und einer Abtriebswelle 15 gelagert sind, die mehrere Zahnräder tragen. Auf der Achse 13 sitzt ein Minutenrad 16, das mit dem Abtrieb eines Gangreglers 17 zusammenwirkt, der durch ein Kästchen veranschaulicht ist, welches die Gangunruhe mit Hemmanker enthält. Ferner sitzt ein kleineres Zahnrad 18 auf der Achse 13, das mit einem größeren Zahnrad 19 auf der Achse 14 eine Zwischenübersetzung (Untersetzungsgetriebe) für die Abtriebswelle 15 bildet. Es sind auch mehr oder weniger Übersetzungen möglich. Die Verbindung zwischen der Achse 14 und der Abtriebswelle 15 erfolgt mit Hilfe kämmender Zahnräder 20 und 21, wobei das Zahnrad 21 auf der Abtriebswelle 15 beträchtlich größer als das Zahnrad 20 auf der Achse 14 ist. Die Abtriebswelle 15 ist von einer Spiralfeder in einem Federhaus 22 umgeben, die die Abtriebswelle 15 gespannt hält, solange sie aufgezogen ist.

Die Abtriebswelle 15 ragt durch die eine Deckplatte 11 hindurch und ist auf ihrem äußeren Teil fest mit einem ersten Zahnrad 23 verbunden. In gleicher Ebene neben dem verhältnismäßig großen ersten Zahnrad 23 ist an der Deckplatte 11 eine Sperrklinke 24 um einen Drehpunkt 26 kippbar befestigt. Die Sperrklinke 24 besteht im wesentlichen aus einem flachen Winkelstück mit einem nach oben gerichteten breiten Arm 27a, von dem rechtwinklig ein schmaler langer Arm 27b ausgeht und aus einem mit dem Arm 27a verbundenen Körper mit einer Keilspitze 25. Der Drehpunkt 26 ist in der unteren Ecke des Armes 27a des Winkelstückes vorgesehen. Die Keilspitze 25 und der lange Arm 27b sind dem Zahnrad 23 zugewandt. Das Winkelstück der Sperrklinke 24 steht unter der Wirkung einer Zugfeder 28, die bei 29 mit dem Winkelstück und bei 30 mit der Deckplatte 11 verbunden ist. Der Angriffspunkt 29 der Zugfeder 28 liegt zwischen dem Drehpunkt 26 der Sperrklinke 24 und dem langen Arm 27b.

Ferner ist an der Deckplatte 11 ein Trägerteil in Form einer Schwinge 31 um einen Drehpunkt 32 schwenkbar gelagert. Die Schwinge 31 ist als rechteckige Platte ausgebildet, die in senkrechter Ausrichtung (Halteposition) mit ihrem einen Längsrand gegen einen von der Deckplatte 11 senkrecht abstehenden Stift 33 anliegt und mit ihrem oberen Randteil 34 eine Abschrägung des langen Armes 27b der Sperrklinke 24 untergreift, wodurch diese in bezug auf das erste Zahnrad 23 in Ausrückstellung gehalten ist. Die Halteposition der Schwinge 31 wird von einer Feder 35 eingestellt, deren eines Ende an eine Seitenkante der Schwinge 31 angreift und deren anderes Ende bei 36 an der Deckplatte 11 befestigt ist. Auf einer Seitenfläche der Schwinge 31 ist ein kleines Zahnrad 37 drehbar gelagert. Das Zahnrad 37 kämmt mit dem Zahnkranz des größeren Zahnrades 23 und wird von diesem in dauernde Drehung versetzt.

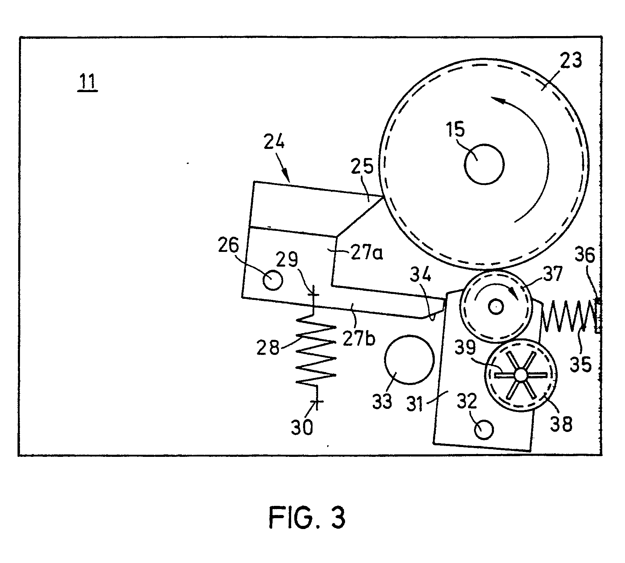

Wenn sich durch einen Fehler in dem Federaufzugsgetriebe, z.B. durch Bruch der Achsen 13 oder 14, die Drehzahl des Zahnrades 23 auf der Abtriebswelle 15 erhöht, baut sich an dem Zahnrad 37 ein Drehmoment auf, welches der Bewegung des Zahnrades 23 entgegenwirkt. Dadurch, daß das Zahnrad 37 auf der Schwinge 31 gelagert ist, versucht diese nun, gegen die Feder 35 auszuweichen. Wenn das Drehmoment an dem Zahnrad 37 so groß geworden ist, daß die Feder 35 gespannt wird, so setzt sich die Schwinge 31 von dem Stift 33 ab und verschwenkt sich um den Drehpunkt 32 in die in Fig. 3 gezeigte Position, in der die Mittelachse des Zahnrades 37 nicht mehr lotrecht unter der Mittelachse des Zahnrades 23 liegt. Die Winkelverstellung der Schwinge 31 gibt den Arm 27b der Sperrklinke 24 frei und diese wird von der Zugfeder 28 angezogen, so daß sie sich um den Drehpunkt 26 dreht und ihre Keilspitze 25 in den Zahnkranz des Zahnrades 23 eingreift. Die Abtriebswelle 15 wird auf diese Weise gegen Verdrehen gesperrt und es ergibt sich eine Sicherung gegen ungewolltes freies Durchlaufen mit unkontrollierter Überfunktion eines anzutreibenden Teiles, z.B. eines Spritzenstößels bei einem Dauerinfusionsgerät.

Auch kann die Funktion der Durchlaufsicherung durch einfaches Zurückdrehen eines Aufziehrades geprüft werden. Da das Funktionsgetriebe durch eine Rutschkupplung mit der Feder verbunden ist, ist es möglich, durch Überwindung des Drehmomentes, welches die Rutschkupplung überträgt, die Durchlaufsicherung von Hand zu aktivieren. Zu diesem Zweck wird mit Hilfe eines nicht gezeigten Aufziehhebels das Zahnrad 23 beschleunigt bzw.

auf solche Drehzahl gebracht, daß ein Einrasten der Sperrklinke 25 erfolgt. Durch Entriegelung wird das Getriebe wieder betriebsbereit gemacht.

Nach Überprüfen der Sicherheitsvorrichtung, bei Neustart des Getriebes oder nach einer Reparatur wird die Sperrklinke 24, z.B. mit Hilfe eines durch die Deckplatte 11 nach außen ragenden Hebels, wieder in ihre Ausrückstellung gebracht, in der der Arm 27b auf dem Rand der Schwinge 31 ruht, die ihre lotrechte Position unter dem Zahnrad 23 wieder eingenommen hat, in der sie von der Feder 35 gegen den Stift 33 gedrückt wird.

Wenn besonders niedrige Abtriebsgeschwindigkeiten vorhanden sind oder bei geringsten Drehzahländerungen der Abtriebswelle 15 gebremst werden soll, wird das Drehmoment an dem Zahnrad 37 durch mindestens ein weiteres Zahnrad 38 erhöht, das kleiner ist als das Zahnrad 37 und zu diesem seitlich versetzt an der Schwinge 31 so angeordnet ist, daß es mit dem Zahnrad 37 kämmt. Zusätzlich zu dem Zahnrad 38 kann auf dessen Drehachse ein Flügelrad 39 angebracht sein, das ebenfalls der Erhöhung der Gegenkräfte an dem Zahnrad 37 dient und geschwindigkeitsabhängig bei höherer Drehzahl bremst.

Ähnlich wie das Flügelrad 39 kann ein Schwungrad an dem Zahnrad 38 vorgesehen sein, dessen Trägheit bei Geschwindigkeitsänderungen sofort einen hohen Widerstand erzeugt. Im übrigen kann das Flügelrad 39 oder ein Schwungrad das Zahnrad 38 ersetzen.

**Ansprüche**

1. Federaufzugsgetriebe für Gas Laufwerk eines Gerätes, insbesondere eines medizinischen Infusionsgerätes, mit einer von einer Spiralfeder gespannten Abtriebswelle (15) und einem Gangregler (17), der über eine Zwischen-Übersetzung auf die Abtriebswelle (15) wirkt, wobei mit der Abtriebswelle (15) ein erstes Kraftübertragungsrad (23) fest verbunden ist, mit dem ein drehbares Sperrelement (24) zusammenwirkt, **dadurch gekennzeichnet,** daß das drehbare Sperrelement (24) von einem beweglichen Trägerteil (31) in Ausrückstellung zu dem ersten Kraftübertragungsrad (23) gehalten ist, und daß an dem Trägerteil (31) ein zweites Kraftübertragungsrad (37) angeordnet ist, das mit dem ersten Kraftübertragungsrad (23) kämmt und das Trägerteil (31) zur Freigabe des Sperrelementes (24) bei erhöhter Drehzahl bzw. Beschleunigung des ersten Kraftübertragungsrades (23) tangential zu diesem auslenkt.

2. Federaufzugsgetriebe nach Anspruch 1,

**dadurch gekennzeichnet,** daß das Sperrelement (24) und Gas Trägerteil (31) federbelastet sind.

3. Federaufzugsgetriebe nach Anspruch 1 oder 2, **dadurch gekennzeichnet,** daß das zweite Kraftübertragungsrad (37) mit mindestens einem Übersetzungselement zusammenwirkt.

4. Federaufzugsgetriebe nach Anspruch 3, **dadurch gekennzeichnet,** daß das Übersetzungselement ein weiteres Kraftübertragungsrad (38) und/ oder ein Flügelrad (39) bzw. Schwungrad aufweist.

5. Federaufzugsgetriebe nach einem der Ansprüche 1 bis 4, **dadurch gekennzeichnet,** daß das Trägerteil (31) als drehbare Schwinge ausgebildet ist.

6. Federaufzugsgetriebe nach einem der Ansprüche 1 bis 4, **dadurch gekennzeichnet,** daß das Trägerteil als tangential zum ersten Kraftübertragungsrad (23) verstellbarer Schlitten ausgebildet ist.

## Claims

1. Spring winding gearing for the drive assembly of a device, especially a medical infusion apparatus, comprising an output shaft (15) tensioned by a coil spring, and a speed controlling means (17) which, through an intermediate transmission, acts upon the output shaft (15), the output shaft (15) having a first power transmission wheel (23) fixedly attached thereto for cooperation with a rotatable locking element (24), **characterized in** that the rotatable locking element (24) is held, by a movable support member (31), in the disengaged position from the first power transmission wheel (23), and in that a second power transmission wheel (37) is arranged at the support member (31), meshing with the first power transmission wheel (23) and, upon increased rotational speed or acceleration of the first power transmission wheel (23), tangentially displacing the support member (31) with respect to the first power transmission wheel (23) to release the locking element (24).

2. Spring winding gearing according to claim 1, characterized in that the locking element (24) and the support member (31) are spring-loaded.

3. Spring winding gearing according to claim 1 or 2, characterized in that the second power transmission wheel (37) cooperates with at least one transmission element.

4. Spring winding gearing according to claim 3, characterized in that the transmission element comprises a further power transmission wheel (38) and/or a fan wheel (39) or a fly wheel, respectively.

5. Spring winding gearing according to any one of claims 1 to 4, characterized in that the support member (31) is provided as a pivotable rocking part.

6. Spring winding gearing according to any one of claims 1 to 4, characterized in that the support member is provided as a sliding carriage which is movable tangentially with respect to the first power transmission wheel (23).

## Revendications

1. Transmission pour un moteur à ressort d'un appareil, en particulier d'un appareil médical d'infusion, comportant un arbre d'entraînement (15) sollicité par un ressort spiral et un régulateur de déplacement (17) qui agit par un transfert intermédiaire sur l'arbre d'entraînement (15), une première roue de transfert de force (23), avec laquelle coopère un élément rotatif de blocage (24), étant reliée de façon fixe à l'arbre d'entraînement (15),
   caractérisée en ce que
   l'élément rotatif de blocage (24) est maintenu par une pièce de support mobile (31) dans une position rétractée par rapport à la première roue de transfert de force (23)
   et en ce qu'il est disposé sur la partie de support (31) une deuxième roue de transfert de force (37) qui engrene avec la première roue de transfert de force (23) et fait dévier la partie de support (31) tangentiellement par rapport à la première roue de transfert de force (23) pour libérer l'élément ce blocage (24) en cas d'accroissement de la vitesse de rotation ou d'accélération de la première roue de transfert de force (23).

2. Transmission de moteur à ressort selon la revendication 1,
   caractérisée en ce que
   l'élément de blocage (24) et la pièce de support (31) sont sollicités élastiquement.

3. Transmission de moteur à ressort selon la revendication 1 ou 2,
   caractérisée en ce que
   la deuxième roue de transfert de force (37) coopère avec au moins un élément de transfert.

4. Transmission de moteur à ressort selon la revendication 3,
   caractérisée en ce que
   l'élément de transfert présente une autre roue de transfert (38) et/ou une roue à ailettes (39) et/ou un volant.

5. Transmission de moteur à ressort selon l'une des revendications 1 à 4,
   caractérisée en ce que
   la partie de support (31) est une bielle rotative.

6. Transmission de moteur à ressort selon l'une des revendications 1 à 4,
   caractérisée en ce que
   la pièce de support est réalisée sous forme de chariot déplaçable tangentiellement par rapport à la première roue de transfert de force (23).

FIG. 1

FIG. 2

FIG. 3